(19) **Europäisches Patentamt**

**European Patent Office**

**Office européen des brevets**

(11) **EP 1 342 708 A1**

(12) **EUROPEAN PATENT APPLICATION**
published in accordance with Art. 158(3) EPC

(43) Date of publication:
**10.09.2003   Bulletin 2003/37**

(51) Int Cl.⁷: **C07C 5/29**, C07C 13/615,
B01J 29/12

(21) Application number: **01270512.5**

(22) Date of filing: **03.12.2001**

(86) International application number:
**PCT/JP01/10548**

(87) International publication number:
**WO 02/048077 (20.06.2002 Gazette 2002/25)**

(84) Designated Contracting States:
**AT BE CH CY DE DK ES FI FR GB GR IE IT LI LU MC NL PT SE TR**

(30) Priority: **11.12.2000   JP   2000375590**
             **12.06.2001   JP   2001176445**

(71) Applicant: **IDEMITSU PETROCHEMICAL CO., LTD.**
**Tokyo 130-0015 (JP)**

(72) Inventors:
• **KOJIMA, Akio**
  **Tokuyama-shi, Yamaguchi 745-0843 (JP)**

• **KODOI, Kouichi**
  **Tokuyama-shi, Yamaguchi 745-0843 (JP)**
• **TSURUTA, Shunji**
  **Kitakyusyu-shi, Fukuoka 808-0027 (JP)**
• **OGATA, Masamitsu**
  **Kawasaki-shi, Kanagawa 212-0013 (JP)**

(74) Representative:
**Gille Hrabal Struck Neidlein Prop Roos Patentanwälte,**
**Brucknerstrasse 20**
**40593 Düsseldorf (DE)**

(54) **PROCESS FOR PRODUCING ADAMANTANE COMPOUND**

(57)     There is provided a process for producing adamantane and analogues thereof, namely a hydrocarbon having an adamantane structure which process comprises isomerizing a tricyclic saturated hydrocarbon having ten or more carbon atoms in the presence of a catalyst in which one or two or more metals selected from among the metals belonging to group VIII in the Periodic Table (group 8 to 10 in the new Periodic Table) are supported on zeolite by means of an ion exchange method. It is made possible by the above process to efficiently produce adamantane and analogues thereof in the presence of a solid catalyst without the use of hydrogen chloride.

# EP 1 342 708 A1

**Description**

TECHNICAL FIELD

The present invention relates to a process for producing a hydrocarbon having an adamantane structure by isomerizing a tricyclic saturated hydrocarbon having ten or more carbon atoms, particularly to a process for efficiently producing adamantane and analogues by the use of a solid catalyst without using hydrogen chloride.

BACKGROUND ART

Adamantane is a compound which is obtained by isomerizing, in the presence of a catalyst, trimethylene norbornane (hereinafter sometimes referred to as "TMN") obtainable by hydrogenating dicyclopentadiene (hereinafter sometimes referred to as "DCPD"). In an industrial process for producing the same, aluminum chloride has heretofore been employed as a catalyst. However, in the case of producing adamantane in the presence of aluminum chloride as a catalyst, it is necessary to use a large amount thereof and besides, the catalyst is not reusable because of complex formation with heavy components during the course of reaction. Accordingly, the foregoing process, when being employed therefor, brings about the formation of a large amount of waste aluminum components, whereby the waste treatment thereof gives rise to a problem of environmental pollution. In addition, high corrosiveness of aluminum chloride necessitates the use of an expensive corrosion-resistant materials of construction. Moreover, aluminum chloride, when used therefor, causes the resultant adamantane to be colored and thereby brings about such disadvantages that recrystallizing step and decolorizing step by means of activated carbon or the like are required and hence, a post treatment is made intricate.

On the other hand, a solid-acid catalyst is known which comprises an active metal such as platinum, rhenium, nickel or cobalt each being supported by impregnation method on zeolite that has been subjected to cation exchange by the use of a rare earth metal or an alkaline earth metal {refer to Japanese Patent Publication No. 2909/1977 (Showa 52)}. However, even in the case where the aforesaid solid-acid catalyst is employed, the yield of adamantane is low, unless hydrogen chloride is allowed to coexist therewith, for instance, conversion of TMN of 79.5%, selectivity to adamantane of 10.1% and yield of adamantane of 8.0%. Therefore, hydrogen chloride is indispensable for the isomerization, but high corrosiveness of hydrogen chloride necessitates the use of an expensive corrosion-resistant materials of construction {(refer to Japanese Patent Publication No. 2909 /1977 (Showa 52)}.

DISCLOSURE OF THE INVENTION

An object of the present invention is to provide a process capable of efficiently producing adamantane and analogues thereof by the use of a solid catalyst instead of hydrogen chloride by solving the above-mentioned problems.

As the result of intensive extensive research and investigation made by the present inventors, it has been found that the above-mentioned object is achieved by carrying out the isomerization reaction in the presence of a catalyst in which at least one metal from among the metals belonging to group VIII in the Periodic Table (group 8 to 10 in the new Periodic Table) is supported on zeolite by means of an ion exchange method. Thus the present invention has been accomplished on the basis of the foregoing findings and information.

Specifically, the present invention provides a process for producing adamantane and analogues thereof, namely a hydrocarbon having an adamantane structure which process comprises isomerizing a tricyclic saturated hydrocarbon having ten or more carbon atoms in the presence of a catalyst in which one or two or more metals selected from among the metals belonging to group VIII in the Periodic Table (group 8 to 10 in the new Periodic Table) are supported on zeolite by means of an ion exchange method.

THE MOST PREFERRED EMBODIMENT TO CARRY OUT THE INVENTION

As mentioned hereinabove, the catalyst to be used in the production process according to the present invention is one or two or more metals which are selected from among the metals belonging to group VIII in the Periodic Table (group 8 to 10 in the new Periodic Table), and which are supported on zeolite by means of an ion exchange method.

The metals belonging to group VIII in the Periodic Table (group 8 to 10 in the new Periodic Table) are not specifically limited. Examples thereof include iron, cobalt, nickel, ruthenium, rhodium, palladium, osmium, iridium and platinum, of which platinum is preferable.

The catalyst to be used in the production process according to the present invention can be produced by bringing any of metals as mentioned above in the state of, for instance, an aqueous solution of a metal salt or a metal complex salt into contact with zeolite so as to subject any of the metals to ion exchange with the cation site (for instance,

$H^+$, $NH4^+$) in the X or Y type zeolite, drying and calcining the zeolite thus ion exchanged. The amount of the one or two or more metals is not specifically limited, but is preferably at least 0.1% by weight.

The catalyst may be of an optional shape such as powder or granule.

The starting material to be used in the production process according to the present invention is a tricyclic saturated hydrocarbon having ten or more carbon atoms, which is specifically exemplified by trimethylene norbornane (tetrahydrodicyclopentadiene); dimethyltrimethylene norbornane; perhydroacenaphthene; perhydrofluorene; perhydrophenalene; 1,2-cyclopentanoperhydronaphthalene; perhydroanthracene, perhydrophenanthrene; and 9-methylperhydroanthracene. The above-exemplified tricyclic saturated hydrocarbon can be produced by a well-known process, for instance, by the hydrogenation of a corresponding unsaturated hydrocarbon.

The isomerization reaction in the production process according to the present invention is carried out in the presence of the above-mentioned catalyst under the conditions including a reaction temperature in the range of 150 to 500°C, preferably 200 to 400°C and reaction pressure of atmospheric pressure or under pressure. The reaction system may be either continuous system or batchwise system. The reaction is preferably carried out in the presence of hydrogen from the viewpoint of enhancement of adamantane yield.

The amount of the catalyst to be used is 0.01 to 2 (weight of catalyst / weight of starting material), preferably 0.05 to 1 (weight of catalyst / weight of starting material) in the case of batchwise system.

The regeneration of the catalyst can be made by a calcining method in air.

In what follows, the present invention will be described in more detail with reference to comparative examples and working examples, which however shall never limit the present invention thereto.

Example 1

Na-form Y type zeolite (hereinafter referred to as "NaY") which had a $SiO_2$ / $Al_2O_3$ molar ratio of 5.0 in an amount of 235 g was suspended by stirring in 2000 g of pure water. To the resultant suspension was added 114 g of ammonium sulfate to dissolve in the suspension and thereafter the mixture was heated to 60°C with stirring for 30 minutes. The resultant slurry was filtered, and then washed by pouring 2500 g of pure water. The washed cake was dried at 110°C overnight, and calcined in air at 600°C for 3 hours to obtain a primary ion exchanged product. The resultant primary ion exchanged product was suspended in 2000 g of pure water. To the resultant suspension was added 228 g of ammonium sulfate to dissolve in the suspension and thereafter the mixture was heated to 95°C with stirring for 30 minutes. Thereafter the suspension was washed with 2000 g of pure water. The foregoing procedure was repeated three times, and the secondary ion exchanged product thus obtained was termed $NH_4$-form Y type zeolite (hereinafter referred to as "$NH_4Y$"). The $NH_4Y$ in an amount of 178 g was placed in a tubular vessel, subjected to steaming at 510°C for 30 minutes in 100% steam, and suspended by stirring in 2000 g of pure water. To the steamed $NH_4Y$ was added 283 g of 25% sulfuric acid over a period of 30 minutes. Then, the resultant NH 4Y slurry was heated to raise the liquid temperature up to 95°C, subjected to an acid treatment for one hour, filtered followed by washing, and dried at 110°C overnight to obtain H-form ultrastable Y type zeolite (hereinafter referred to as "HUSY") which had a lattice constant of 24.47 and a $SiO_2$ / $Al_2O_3$ molar ratio as obtained from Breck's formula of 10.4. The HUSY thus obtained in an amount of 170 g was suspended by stirring in 2000 g of pure water. To the resultant suspension was added 180 g of 1.71% aqueous solution of tetraammineplatinum chloride with stirring at 60°C for 30 minutes. The resultant mixed suspension was filtered, washed, and dried at 110°C overnight to obtain a 0.93% Pt /HUSY.

An autoclave having an internal volume of 100 milliliter (mL) was charged with the catalyst in an amount of 8 g which had been obtained through the foregoing procedure and 40 g of trimethylene norbornane (TMN), and pressurized inside with hydrogen up to 2 MPa. The content in the autoclave was heated to raise the temperature thereof from room temperature to 250°C for a period of 2 hours, and after the temperature reached 250°C, the reaction was put into practice for two hours. The reaction results are given in Table 1.

In this connection, conversion of TMN and selectivity to adamantane were each calculated by the following formula, respectively:

Conversion of TMN = (1 - weight of TMN after reaction / weight of TMN

before reaction) $\times$ 100

Selectivity to adamantane = {weight of formed adamantane / (weight of

TMN before reaction - weight of TMN after reaction)} $\times$ 100

Comparative Example 1

NaY which had a $SiO_2 / Al_2O_3$ molar ratio of 5.0 in an amount of 235 g was suspended by stirring in 2000 g of pure water. To the resultant suspension was added 114 g of ammonium sulfate to dissolve in the suspension and thereafter the mixture was heated to 60°C with stirring for 30 minutes. The resultant slurry was filtered, and then washed by pouring 2500 g of pure water. The washed cake was dried at 110°C overnight, and calcined in air at 600°C for 3 hours to obtain a primary ion exchanged product. The resultant primary ion exchanged product was suspended in 2000 g of pure water. To the resultant suspension was added 228 g of ammonium sulfate to dissolve in the suspension and thereafter the mixture was heated to 95°C with stirring for 30 minutes. Thereafter the suspension was washed with 2000 g of pure water. The foregoing procedure was repeated three times, and the secondary ion exchanged product thus obtained was termed $NH_4Y$. The $NH_4Y$ in an amount of 178 g was placed in a tubular vessel, subjected to steaming at 510°C for 30 minutes in 100% steam, and suspended by stirring in 2000 g of pure water. To the steamed $NH_4Y$ was added 283 g of 25% sulfuric acid over a period of 30 minutes. Then, the resultant NH4Y slurry was heated to raise the liquid temperature up to 95°C, subjected to an acid treatment for one hour, filtered followed by washing, and dried at 110°C overnight to obtain HUSY which had a lattice constant of 24.47 and a $SiO_2 / Al_2O_3$ molar ratio as obtained from Breck's formula of 10.4.

An aqueous solution was prepared by dissolving 0.1625 g of $Pt(NH_3)_4Cl_2 \cdot H_2O$ (tetraammineplatinum chloride) in 4 mL of pure water. To 10 g of the HUSY thus obtained as the catalyst with kneading was gradually added the aqueous solution of tetraammineplatinum chloride. After the addition of whole amount of the aqueous solution, the catalyst was dried at 130°C for 12 hours and further was calcined at 300°C for 3 hours in a stream of air (supporting by means of pore filling method with a supported amount of Pt of 0.9% by weight).

Subsequently the procedure in Example 1 was repeated to proceed with the reaction except that use was made of the catalyst obtained in the foregoing manner. The reaction results are given in Table 1.

Comparative Example 2

In a short neck flask having an internal volume of 200 mL was placed 10 g of HUSY as the catalyst which had been prepared by the above-mentioned method. To the catalyst therein was added an aqueous solution of 0.1625 g of $Pt(NH_3)_4Cl_2 \cdot H_2O$ dissolved in 100 mL of pure water, followed by stirring for 2 hours. After the completion of the stirring, water was distilled away at 80°C by means of a rotary evaporator. The powdery catalyst thus obtained was dried at 130°C for 12 hours and further was calcined at 300°C for 3 hours in a stream of air (supporting by means of evaporation to dryness method with a supported amount of Pt of 0.9% by weight).

Subsequently the procedure in Example 1 was repeated to proceed with the reaction except that use was made of the catalyst obtained in the foregoing manner. The reaction results are given in Table 1.

Table 1

| | Conversion of TMN (%by weight) | Selectivity to Adamantane (% by weight) | Comp: Comparative |
|---|---|---|---|
| | | | Adamantane yield (% by weight) |
| Example 1 | 96.1 | 20.6 | 19.8 |
| Comp/Example 1 | 75.4 | 16.7 | 12.8 |
| Comp/Example 2 | 63.5 | 14.6 | 9.3 |

Example 2

NaY which had a $SiO_2 / Al_2O_3$ molar ratio of 5.0 in an amount of 235 g was suspended by stirring in 2000 g of pure water. To the resultant suspension was added 114 g of ammonium sulfate to dissolve in the suspension and thereafter the mixture was heated to 60°C with stirring for 30 minutes. The resultant slurry was filtered, and then washed by pouring 2500 g of pure water. The washed cake was dried at 110°C overnight, and calcined in air at 600°C for 3 hours to obtain a primary ion exchanged product. The resultant primary ion exchanged product was suspended in 2000 g of pure water. To the resultant suspension was added 228 g of ammonium sulfate to dissolve in the suspension and thereafter the mixture was heated to 95°C with stirring for 30 minutes. Thereafter the suspension was washed with 2000 g of pure water. The foregoing procedure was repeated three times, and the secondary ion exchanged product thus obtained was termed $NH_4Y$. The resultant $NH_4Y$ was calcined at 500°C for 3 hours to obtain HY. The HY thus obtained was suspended by stirring in 2000 g of pure water. To the resultant suspension was added 180 g of 1.71% aqueous solution of tetraammineplatinum chloride with stirring at 60°C for 30 minutes. The resultant mixed suspension

EP 1 342 708 A1

was filtered, washed, and dried at 110°C overnight to obtain a 0.88% Pt / HY.

The catalyst in an amount of 4 g which had been obtained through the foregoing procedure was packed in a tubular reactor made of stainless steel (SUS), and was calcined at 300°C for 3 hours under atmospheric pressure in a stream of air. After the atmosphere in the reactor was replaced with nitrogen, the catalyst was reduced with hydrogen at 300°C for 3 hours under atmospheric pressure in a stream of hydrogen.

Thereafter, supply of TMN and hydrogen to the reactor was commenced so as to proceed with continuous isomerization reaction under the reaction conditions of 250°C, 2 MPa, weight hourly space velocity (WHSV) being 2.4 $h^{-1}$ (on TMN basis) and hydrogen / TMN molar ratio being 2. The reaction results after 50 hours from the start of TMN supply are given in Table 2.

Comparative Example 3

NaY which had a $SiO_2$ / $Al_2O_3$ molar ratio of 5.0 in an amount of 235 g was suspended by stirring in 2000 g of pure water. To the resultant suspension was added 114 g of ammonium sulfate to dissolve in the suspension and thereafter the mixture was heated to 60°C with stirring for 30 minutes. The resultant slurry was filtered, and then washed by pouring 2500 g of pure water. The washed cake was dried at 110°C overnight, and calcined in air at 600°C for 3 hours to obtain a primary ion exchanged product. The resultant primary ion exchanged product was suspended in 2000 g of pure water. To the resultant suspension was added 228 g of ammonium sulfate to dissolve in the suspension and thereafter the mixture was heated to 95°C with stirring for 30 minutes. Thereafter the suspension was washed with 2000 g of pure water. The foregoing procedure was repeated three times, and the secondary ion exchanged product thus obtained was termed $NH_4Y$. The resultant $NH_4Y$ was calcined at 500°C for 3 hours to obtain HY.

An aqueous solution was prepared by dissolving 0.1625 g of $Pt(NH_3)_4Cl_2 \cdot H_2O$ (tetraammineplatinum chloride) in 4 mL of pure water. To 10 g of the HY thus obtained as the catalyst with kneading was gradually added the aqueous solution of tetraammineplatinum chloride. After the addition of whole amount of the aqueous solution, the catalyst was dried at 130°C for 12 hours and further was calcined at 300°C for 3 hours in a stream of air (supporting by means of pore filling method with a supported amount of Pt of 0.9% by weight).

Subsequently the procedure in Example 2 was repeated to proceed with the reaction except that use was made of the catalyst obtained in the foregoing manner. The reaction results after 50 hours from the start of TMN supply are given in Table 2.

Comparative Example 4

In a short neck flask having an internal volume of 200 mL was placed 10 g of HY as the catalyst which had been prepared by the above-mentioned method. To the catalyst therein was added an aqueous solution of 0.1625 g of $Pt(NH_3)_4Cl_2 \cdot H_2O$ dissolved in 100 mL of pure water, followed by stirring for 2 hours. After the completion of the stirring, water was evaporated away at 80°C by means of a rotary evaporator. The powdery catalyst thus obtained was dried at 130°C for 12 hours and further was calcined at 300°C for 3 hours in a stream of air (supporting by means of evaporation to dryness method with a supported amount of Pt of 1% by weight).

Subsequently the procedure in Example 2 was repeated to proceed with the reaction except that use was made of the catalyst obtained in the foregoing manner. The reaction results are given in Table 2.

Table 2

|  | Conversion of TMN(%by weight) | Selectivity to Adamantane(%by weight) | Adamantane yield(% by weight) |
|---|---|---|---|
| Example 2 | 52.6 | 13.3 | 7.0 |
| Comp/Example 3 | 33.9 | 10.2 | 3.5 |
| Comp/Example 4 | 22.1 | 8.8 | 1.9 |

Example 3

NaY which had a $SiO_2$ / $Al_2O_3$ molar ratio of 5.0 in an amount of 235 g was suspended by stirring in 2000 g of pure water. To the resultant suspension was added dilute nitric acid to adjust the pH of the suspended slurry to 5.5. Aside therefrom, 246 g of lanthanum nitrate hexahydrate was dissolved in 500 g of warm water. The aqueous solution of lanthanum nitrate thus obtained was gradually mixed with the suspended slurry. Thereafter, the resultant mixture was heated to 90°C, stirred for 30 minutes, then filtered and washed. The washed cake was dried at 110°C overnight, and calcined at 600°C for 3 hours.

The powder was again suspended by stirring in 2000 g of pure water, to the resultant slurry was added 228 g of ammonium sulfate, and the mixture was heated to 95°C with stirring for 30 minutes, filtered and washed. The washed cake was again suspended in 2000 g of pure water, and the suspended slurry was subjected to an ion exchange operation twice consecutively. Subsequently, the resultant ion exchanged product was dried at 110°C overnight. The dried product was placed in a tubular vessel, subjected to steaming at 510°C for 30 minutes in 100% steam, and the steamed powder thus obtained was suspended in 2000 g of pure water. The suspended slurry was gradually incorporated with 32 g of 25% sulfuric acid, heated at 95°C for 30 minutes, then filtered and washed. The washed cake was again suspended in 2000 g of pure water. To the resultant suspension was added 180 g of 1. 71% aqueous solution of tetraammineplatinum chloride with stirring at 60°C for 30 minutes. The resultant mixed suspension was filtered, washed, and dried at 110°C overnight to obtain a La-containing zeolite of type USY on which 0.87% platinum was supported by means of ion exchange.

Subsequently, the procedure in Example 2 was repeated to proceed with the reaction except that use was made of the catalyst obtained in the foregoing manner and that the reaction temperature and reaction pressure were set on 325°C and 5 MPa, respectively. The reaction results after 50 hours from the start of TMN supply are given in Table 3.

Comparative Example 5

NaY which had a $SiO_2$ / $Al_2O_3$ molar ratio of 5.0 in an amount of 235 g was suspended by stirring in 2000 g of pure water. To the resultant suspension was added dilute nitric acid to adjust the pH of the suspended slurry to 5.5. Aside therefrom, 246 g of lanthanum nitrate hexahydrate was dissolved in 500 g of warm water. The aqueous solution of lanthanum nitrate thus obtained was gradually mixed with the suspended slurry. Thereafter, the resultant mixture was heated to 90°C, stirred for 30 minutes, then filtered and washed. The washed cake was dried at 110°C overnight, and calcined at 600°C for 3 hours.

The powder was again suspended by stirring in 2000 g of pure water, to the resultant slurry was added 228 g of ammonium sulfate, and the mixture was heated to 95°C with stirring for 30 minutes, filtered and washed. The washed cake was again suspended in 2000 g of pure water, and the suspended slurry was subjected to an ion exchange operation twice consecutively. Subsequently, the resultant ion exchanged product was dried at 110°C overnight. The dried product was placed in a tubular vessel, subjected to steaming at 510°C for 30 minutes in 100% steam, and the steamed powder thus obtained was suspended in 2000 g of pure water. The suspended slurry was gradually incorporated with 32 g of 25% sulfuric acid, heated at 95°C for 30 minutes, then filtered and washed. Then the washed cake was dried at 110°C overnight to obtain a La-containing zeolite type USY. An aqueous solution was prepared by dissolving 0.1265 g of $Pt(NH_3)_4Cl_2 \cdot H_2O$ in 4 mL of pure water. To 10 g of the La-containing zeolite type USY thus obtained as the catalyst with kneading was gradually added the aqueous solution of tetraammineplatinum chloride. After the addition of whole amount of the aqueous solution, the catalyst was dried at 130°C for 12 hours and further was calcined at 300°C for 3 hours in a stream of air (supporting by means of pore filling method with a supported amount of Pt of 0.9% by weight).

Subsequently the procedure in Example 3 was repeated to proceed with the reaction except that use was made of the catalyst obtained in the foregoing manner. The reaction results after 50 hours from the start of TMN supply are given in Table 3.

Example 4

The procedure in Example 3 was repeated to proceed with the reaction by the use of the catalyst obtained in Example 3 except that the reaction temperature was set on 350°C. The reaction results after 50 hours from the start of TMN supply are given in Table 3.

Comparative Example 6

The procedure in Comparative Example 5 was repeated to proceed with the reaction by the use of the catalyst obtained in Comparative Example 5 except that the reaction temperature was set on 350°C. The reaction results after 50 hours from the start of TMN supply are given in Table 3.

Table 3

|  | Reaction Temperature (°C) | Conversion of TMN (% by weight) | Selectivity to Adamantane (% by weight) | Adamantane yield (% by weight) |
|---|---|---|---|---|
| Example 3 | 325 | 91,2 | 15.3 | 14.0 |
| Comp/Example 5 | 325 | 65.4 | 13.8 | 9.0 |
| Example 4 | 350 | 95.2 | 12.8 | 12.2 |
| Comp/Example 6 | 350 | 70.4 | 10.1 | 7.1 |

INDUSTRIAL APPLICABILITY

According to the present invention, by carrying out the isomerization reaction of a tricyclic saturated hydrocarbon in the presence of a catalyst in which one or two or more metals selected from among the metals belonging to group VIII in the Periodic Table (group 8 to 10 in the new Periodic Table) are supported on zeolite by means of an ion exchange method, it is made possible to markedly enhance the yield of adamantane and analogues thereof as compared with the case where the same metal or metals are supported thereon by means of an impregnation method such as pore filling method or evaporation to dryness method. In addition, since no use is made of a highly corrosive substance such as hydrogen chloride at the time of production, it is made possible to efficiently produce adamantane and analogues thereof at a low cost, dispensing with the use of a corrosion-resistant material in production equipment.

**Claims**

1.   A process for producing adamantane and analogues thereof, namely a hydrocarbon having an adamantane structure which process comprises isomerizing a tricyclic saturated hydrocarbon having ten or more carbon atoms in the presence of a catalyst in which one or two or more metals selected from among the metals belonging to group VIII in the Periodic Table (group 8 to 10 in the new Periodic Table) are supported on zeolite by means of an ion exchange method.

2.   The process for producing adamantane and analogues thereof according to Claim 1, wherein the tricyclic saturated hydrocarbon having ten or more carbon atoms is selected from the group consisting of trimethylene norbornane (tetrahydrodicyclopentadiene); dimethyltrimethylene norbornane; perhydroacenaphthene; perhydrofluorene; perhydrophenalene; 1,2-cyclopentanoperhydronaphthalene; perhydroanthracene, perhydrophenanthrene; and 9-methylperhydroanthracene.

3.   The process for producing adamantane and analogues thereof according to Claim 1, wherein the metal belonging to group VIII in the Periodic Table (group 8 to 10 in the new Periodic Table) is platinum.

4.   The process for producing adamantane and analogues thereof according to Claim 1, wherein the zeolite is Y type zeolite.

5.   The process for producing adamantane and analogues thereof according to Claim 1, wherein the amount of the metal or metals that are supported on the zeolite is at least 0.1% by weight based on the amount of the catalyst.

6.   The process for producing adamantane and analogues thereof according to Claim 1, wherein the isomerization is effected at a reaction temperature of 150 to 500°C, and a reaction pressure of atmospheric or increased pressure.

7.   The process for producing adamantane and analogues thereof according to Claim 1, wherein the isomerization is effected at a reaction temperature of 200 to 400°C, and a reaction pressure of atmospheric or increased pressure.

## INTERNATIONAL SEARCH REPORT

| International application No. |
| --- |
| PCT/JP01/10548 |

| A. CLASSIFICATION OF SUBJECT MATTER |
| --- |
| Int.Cl⁷ C07C5/29, 13/615, B01J29/12 |
| According to International Patent Classification (IPC) or to both national classification and IPC |

| B. FIELDS SEARCHED |
| --- |
| Minimum documentation searched (classification system followed by classification symbols)<br>Int.Cl⁷ C07C5/27-5/31, 13/615, B01J29/12 |
| Documentation searched other than minimum documentation to the extent that such documents are included in the fields searched |
| Electronic data base consulted during the international search (name of data base and, where practicable, search terms used) |

| C. DOCUMENTS CONSIDERED TO BE RELEVANT | | |
| --- | --- | --- |
| Category* | Citation of document, with indication, where appropriate, of the relevant passages | Relevant to claim No. |
| X<br>Y | JP 2-6855 A (Kawasaki Steel Corporation),<br>11 January, 1990 (11.01.1990) (Family: none) | 1,2,4-7<br>3 |
| Y | US 3944626 A (HONNA, Kosaku; SHIMIZU, Nobuaki;<br>KURISAKI, Konomu),<br>16 March, 1976 (16.03.1976)<br>& JP 49-133362 A    & JP 50-32154 A<br>& DE 2419620 A1    & FR 2227247 A1<br>& GB 1407960 A    & CH 599078 A | 3 |
| Y | JP 60-246333 A (Idemitsu Kosan Co., Ltd.),<br>06 December, 1985 (06.12.1985) (Family: none) | 3 |

☐ Further documents are listed in the continuation of Box C.  ☐ See patent family annex.

| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |
| Date of the actual completion of the international search<br>29 January, 2002 (29.01.02) | Date of mailing of the international search report<br>12 February, 2002 (12.02.02) |
| Name and mailing address of the ISA/<br>Japanese Patent Office | Authorized officer |
| Facsimile No. | Telephone No. |

Form PCT/ISA/210 (second sheet) (July 1992)